# EUROPEAN PATENT APPLICATION

(11) **EP 1 371 374 A1**
(43) Date of publication of application: **17.12.2003**
(21) Application number: 02012844.3
(22) Date of filing: 10.06.2002
(51) Int. Cl.: A61K 39/00, A61K 39/29, A61K 39/39, A61P 37/04

(54) **Immunotherapy of chronic infections and of malignant diseases**

(71) Applicant: Endres, Stefan, Prof. Dr., 80805 München (DE)
(72) Inventor: Endres, Stefan, Prof. Dr., 80805 München (DE)
(74) Representative: Weiss, Wolfgang, Dipl.-Chem. Dr.

(57) **Abstract**

The present invention relates to methods and compositions for the treatment of chronic infections or malignant diseases, particularly chronic, interferon-resistant viral infections such as hepatitis B infections. Further, the present invention relates to methods and compositions for the improvement of interferon-α therapies.

## Description

### 1. Field of the Invention

The present invention relates to methods and compositions for the treatment of chronic infections or malignant diseases, particularly chronic, interferon-resistant viral infections such as hepatitis B infections. Further, the present invention relates to methods and compositions for the improvement of interferon-α therapies.

### 2. Hepatitis B: Natural History

Hepatitis B is an infection primarily of the liver with the hepatitis B virus (HBV). It occurs perinatally (from the mother to the child), through sexual contact, through contact with infectious blood, e.g. during i.v. drug abuse or blood transfusions. In about one third of the patients the mode of infection cannot be identified.

After infection the disease can be limited (e.g. elimination of the virus within less than six months; i.e. acute hepatitis B). If the infection persists for more than six months it is called chronic hepatitis B infection.

Chronic hepatitis B infection rarely (5 to 10% per year) resolves (i.e. clearance of virus) spontaneously. When chronic hepatitis B infection persists for many years fibrosis and cirrhosis of the liver can develop as complications. Cirrhosis develops in approximately 30% of patients after a mean of 20 years of infection. In those patients with cirrhosis as a complication of chronic hepatitis B there is a 3 to 5% annual risk of developing hepatocellular carcinoma. This is a malignant, often incurable disease.

### 3. Hepatitis B: Treatment

The approved treatment options for chronic hepatitis B are interferon-α and lamivudine. Interferon-α is a cytokine (protein) with directly antiviral and, indirect, immunostimulatory activities. Other approved indications for interferon-α are hairy-cell leukemia, chronic myeologenic leukemia, and AIDS-related Kaposi's sarcoma. Further indications, presently under investigation, are adjuvant therapy for malignant melanoma, AIDS-related thrombocytopenia, cutaneous ulcerations of Behcet's disease, brain tumors, metastatic ileal carcinoid tumors, cervical and colorectal cancers, genital warts, idiopathic mixed cryoglobulinemia, hemangioma, hepatitis D, hepatocellular carcinoma, idiopathic hypereosinophilic syndrome, mycosis fungoides, Sezary syndrome, low-grade non-Hodgkin's lymphoma, macular degeneration, multiple myeloma, renal cell carcinoma, basal and squamous cell skin cancer, essential thrombocythemia and cutaneous T-cell lymphoma. For the treatment of hepatitis B, interferon-α is given three times per week by subcutaneous injection, usually for a period of four to six months. Lamivudine is a nucleoside analogue which is given orally, daily, for extended time, from one to four years. The surrogate marker for treatment success for both treatments is the elimination of replicating virus from the body. This can be measured by loss of detectable HBV-DNA (by polymerase chain reaction) and by loss of hepatitis B antigen e (HBe Ag) (mostly concurrent with development of anti-HBe antibodies) in the serum.

Therapy with interferon-α leads to normalisation of liver enzymes and loss of viral DNA at the end of the treatment (primary response) in about 50% of patients. Of these 10% relapse, i.e. replicating virus reccurs within one year after interferon-α treatment is discontinued. Thus about 40% remain free of detectable viral DNA one year after end of treatment. This is defined as a sustained response.

The optimal treatment for those 60 percent of patients who do not respond (no primary response or relapse after primary response) to interferon-α is unknown and no medication is approved for this situation. Mostly the patients are observed. Experimental regimens are a second course of interferon, lamivudine, combination of interferon with the nucleoside analogon ribaririn or new antiviral agents.

Chronic hepatitis B is an immense global health problem. It affects 300 million individuals or 5 percent of the world's population. Through its complication hepatocellular carcinoma it is the leading cause of cancer in humans.

Treatment with interferon-α is only effective in about 50% of the patients (as outlined above). It is expensive (5000 Euro per six months treatment course) and carries frequent, partially severe side effects.

### 4. Case Observation

Surprisingly it was found that an unrelated vaccination may improve the efficiency of therapies against chronic infections, particularly chronic viral infections. The term "unrelated vaccination" in this context relates to a vaccination against a pathogen which is not related to the pathogen causing the chronic infection. More particularly, the immune system does not exhibit a substantial cross-reactivity against the pathogen associated with the chronic infection and the pathogen which is the target of the unrelated vaccination.

### 5. Description of the Invention

Thus, a first subject matter of the invention is a method for the treatment of a chronic infection or a malignant disease comprising co-administering an anti-pathogenic agent and/or immunostimulatory agent for the treatment of a chronic infection or a malignant disease, and an unrelated vaccination. The chronic infection is particularly a chronic viral or fungal infection, more particularly a hepatitis infection, e.g. a hepatitis B or hepatitis C infection.

Other preferred types of infections which may be treated with the method of the invention are other chronic viral infections such as by HIV, herpes- or papillomavirus, bacterial infections such as by Mycobacterium leprae, protozoal infections such as Leishmaniasis, fungal infections such as Aspergillosis or helminthic infections such as Schistosomiasis. The malignant disease is particularly a disease which may be treated by administration of an interferon, particularly interferon-α, e.g. as described above.

The unrelated vaccination is unrelated to the existing infection or malignant disease. The unrelated vaccination may comprise the administration of an antigen and/or an adjuvant, wherein the antigen is capable of eliciting a specific immune response against an unrelated disease and the adjuvant is capable of elicting an unspecific stimulation of the immune system. By administering the unrelated vaccination of the present invention, the efficiency of the anti-pathogenic and/or immunostimulatory treatment is increased, which may even lead to a complete elimination of the pathogen causing the chronic infection. The agent which is co-administered with the unrelated vaccination is preferably an antiviral and/or immunostimulatory agent, more preferably an interferon and most preferably interferon-α, e.g. recombinant interferon-α or pegylated interferon-α.

The chronic infection which is treated by the method of the invention is particularly a chronic infection which is at least partially resistant to the treatment of an antipathogenic agent. More particularly, the chronic infection is an interferon-resistant hepatitis infection. By co-administration of the unrelated vaccination, the efficiency of the anti-pathogenic treatment is increased.

The malignant disease treated by the method of the invention is particularly a malignant disease which is subject to a treatment with interferon optionally in combination with other modes of treatment, preferably administration of cytostatic or cytotoxic agents and/or tumor vaccination. This tumor vaccination may comprise the administration of allogeneic or autologous tumor cells or components thereof, e.g. tumor cell lysates, tumor-associated antigens or peptides derived from such antigens. For example, a tumor patient is treated with interferon, e.g. interferon-α and a tumor vaccination; and an unrelated vaccine is coadministered simultaneously, overlapping or preferably sequentially.

The unrelated vaccination preferably comprises a vaccination against a bacterial infection, e.g. a vaccination against diphtheria, tetanus, pertussis, or a viral infection, e.g. a vaccination against influenza, etc. The vaccine preferably comprises a bacterial toxoid such as diphtheria toxoid, tetanus toxoid, or pertussis toxoid.

In a further embodiment, the unrelated vaccination comprises the administration of adjuvant alone, e.g. aluminum hydroxide, aluminum phosphate etc., or in combination with an antigen, e.g. as described above.

The unrelated vaccination may comprise a single vaccination step or preferably a plurality of vaccination steps, particularly a priming step and at least one and preferably several booster steps. The unrelated vaccination may be carried out by usual protocols as known in the art.

The method of the present invention comprises a co-administration of an anti-pathogenic and/or immunostimulatory agent and an unrelated vaccination. The co-administration comprises administering both components in the same patient simultaneously, in an overlapping schedule or sequentially. A simultaneous or overlapping co-administration means that the vaccination or at least one vaccination step is carried out while the patient to be treated is under treatment with an anti-pathogenic and/or immunostimulatory agent. A sequential co-administration comprises that there is a predetermined time interval between the anti-pathogenic and/or immunostimulatory treatment and the unrelated vaccination. This time interval may be, e.g. from one day to six months. Especially preferred is an embodiment of sequential co-administration comprising an initial anti-pathogenic and/or immunostimulatory, e.g. interferon treatment and a subsequent unrelated vaccination, preferably comprising a plurality of vaccination steps. The time period between the end of the initial anti-pathogenic and/or immunostimulatory treatment and the start of the unrelated vaccination is preferably between one day and six months more preferably between one day and 30 days and most preferably between 5 and 15 days.

A further subject-matter of the present invention is an improvement of interferon-α-therapy, particularly for the treatment of malignant diseases, viral infections or fungal infections, e.g. interferon-α applications as described above, comprising co-administering an unrelated vaccination, preferably an unrelated vaccination against an infectious disease, more preferably a bacterial disease.

Still a further subject matter of the invention is a method for the treatment of a chronic interferon-resistant viral infection comprising administering an unrelated vaccination, preferably a diphtheria vaccination, wherein the vaccine comprises a bacterial toxoid and/or an adjuvant. Preferably, this method additionally comprises administering an interferon, more particularly interferon-α. For further preferred embodiments of this aspect of the invention, it is referred to the discussion above.

Still a further subject matter of the invention is a composition or kit for the treatment of infections or malignant diseases, comprising:
- an anti-pathogenic agent and/or immunostimulatory agent, particularly interferon-α, and
- a vaccine against an unrelated disease.

Still a further embodiment of the present invention relates to a composition or kit for the treatment of a chronic interferon-resistant viral infection comprising:
- a vaccine against an unrelated disease comprising a bacterial toxoid and/or an adjuvant.

Further, the compositions or kits may comprise usual pharmaceutically acceptable carriers, diluents and adjuvants.

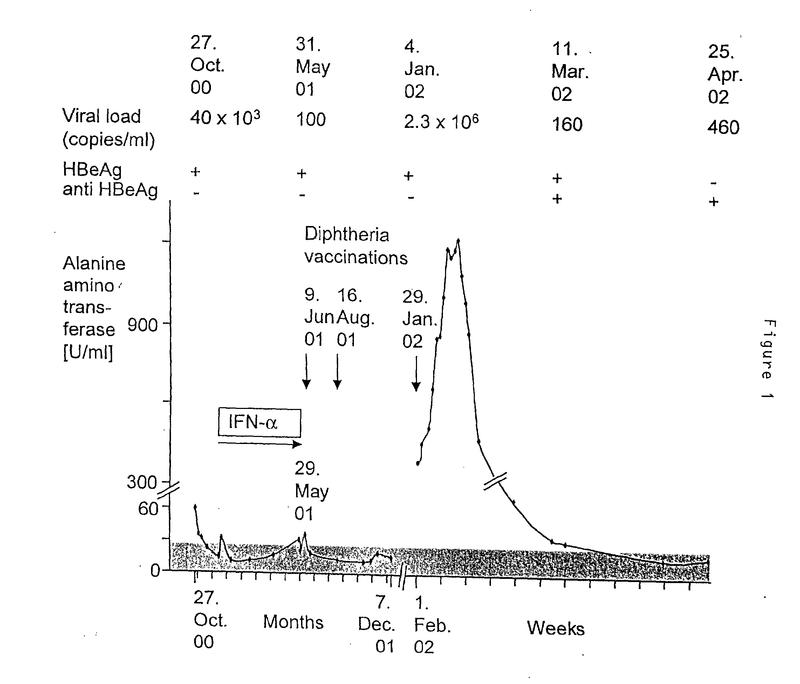

Moreover, the invention shall be explained by the following example in combination with Figure 1.

Figure 1 shows a preferred treatment protocol according to the present invention.

### 6. Example

Acute flare-up of chronic hepatitis B infection and subsequent HBeAg seroconversion associated with diphtheria vaccination

In a 36-year old asymptomatic native of Vietnam living in Germany for 20 years chronic hepatitis B infection was diagnosed in December 2000. The same diagnosis in his brother, growing up in an endemic area and the absence of other risk markers, especially illicit drug abuse, suggested a connatal or early childhood infection. His alanine amino transferase (ALT) level was 60 U/ml, HBsAg and HBeAg were positive, 40.000 hepatitis B viral copies/ml were detected in quantitative PCR and liver histology showed minimal fibrosis, minimal steatosis and only few lymphocytes in one portal field. Therapy with interferon-α2b 3 x 5 million units/week, initiated on December 16th, was well tolerated and ALT levels were normalised within three weeks (Fig. 1). In May 2001 (after six months of therapy) the patient presented with tachycardia and tremor. Thyrotoxicosis was diagnosed with suppressed thyrotropin and elevated thyroxin to 7.4 ng/ml (normal up to 4.3 ng/ml). Homogeneous thyroid translucency on ultrasound and positive anti-thyrotropin-receptor-antibodies suggested autoimmune thyroiditis, probably induced by interferon-α therapy. At that time, hepatitis B virus PCR was still positive with 100 copies/ml. Interferon-α-therapy was discontinued and thyrotoxicosis was well controlled with carbimazol.

On February 1st 2002, at a routine visit the patient was asymptomatic but an ALT level of 387 U/ml was found. Three days earlier the patient had received a third dose of diphtheria vaccine (0.5 ml subcutaneously; Diphtherie-Adsorbat-Behring®, Chiron-Behring GmbH, Marburg, Germany) with previous equal injections five and seven months earlier. Carbimazol had already been tapered to 5 mg/dl and blood tests gave no evidence for autoimmune hepatitis or metabolic liver disease. The hepatitis-B virus load had increased to 2.2 million copies/ml and liver histology showed medium grade portal inflammation with regions of increased cellular infiltrates and slight intralobular inflammatory reaction. A tentative diagnosis of an acute flare of chronic hepatitis B was made, possibly precipitated by the vaccination. Acute flare often precedes seroconversion indicating a complete elimination of virus (Hoofnagle et al., Ann. Intern. Med. 1981; 94:744; Realdi et al. Gastroenterology 1980; 79: 195; Alward et al., J. Infect. Dis. 1985; 151: 604). Carbimazol was discontinued. No other treatment was instituted. ALT levels peaked within eight days at 1200 U/ml, aspartate aminotransferase (AST) at 365 U/ml and bilirubin at 5.7 mg/dl [97 µM]. The flare-up of hepatitis had spontaneously resolved six weeks later (normal ALT). At that time the viral load had descreased to 160 copies/ml and, for the first time, anti-HBe antibodies were detected (with continued presence of HBe antigen). Another five weeks later HBeAg was no longer detectable. Reexposure to carbimazol without any change in liver function tests ruled out carbimazol toxicity as a cause of the exacerbation.

The close temporal association, the previous long-term (over one-and-a half years) documentation of normal or maximally three-fold elevated ALT levels, and exclusion of other causes suggests diphtheria vaccination as the possible cause precipitating the flareup and subsequent HBe seroconversion. Search of the databases and inquiry with the manufacturer revealed that no case of chronic hepatitis B exacerbation after vaccination with diphtheria toxoid has been reported.

The effect of diphtheria vaccination in a patient with chronic hepatitis B who had not seroconverted under standard interferon-α therapy must be regarded as completely surprising.

## Claims

1. A method for the manufacture of a medicament for the treatment of a chronic infection and/or a malignant disease comprising formulating a composition or kit comprising an anti-pathogenic and/or immunostimulatory agent and a vaccine against an unrelated disease.

2. The method of claim 1, wherein said chronic infection is a viral infection and the anti-pathogenic and/or immunostimulatory agent is an anti-viral agent.

3. The method of claim 1 or 2, wherein said chronic infection is a hepatitis infection, particularly a hepatitis B infection.

4. The method of any one of claims 1-3, wherein said anti-pathogenic and/or immunostimulatory agent comprises an interferon-α.

5. The method of anyone of claims 1-4 wherein composition or kit further comprises an anti-tumor vaccine.

6. The method of any one of claims 1-5, wherein said unrelated vaccine is a vaccine against a bacterial infection, particularly against diphtheria.

7. The method of any one of claims 1-6, wherein said unrelated vaccine is a multi-dosage vaccine.

8. The method of any one of claims 1-7, wherein said composition or kit is co-administered is simultaneously in an overlapping schedule and/or sequentially.

9. The method of claim 8, wherein said sequential co-administering comprises an initial anti-pathogenic and/or immunostimulatory treatment and a subsequent unrelated vaccination.

10. The method of claim 9, wherein the time period between the end of the anti-pathogenic and/or immunostimulatory treatment and the start of the unrelated vaccination is between one day and six months.

11. A method for the manufacture of a medicament for the improvement of an interferon-α therapy comprising formulating a composition or kit comprising interferon-α and a vaccine against an unrelated disease.

12. A method for the manufacture of a medicament for the treatment of interferon-resistant chronic viral infection comprising formulating a composition or kit comprising a vaccine against an unrelated disease wherein said vaccine comprises a bacterial toxoid and/or an adjuvant.

13. The method of claim 12, comprising additionally administering an interferon.

14. A composition or kit for the treatment of chronic infections and/or malignant diseases comprising:
- an anti-pathogenic agent and/or immunostimulatory agent and
- a vaccine against an unrelated disease.

15. A composition or kit for the treatment of interferon-resistant chronic viral infection comprising:
- a vaccine against an unrelated disease comprising a bacterial toxoid and/or an adjuvant.
